# EUROPÄISCHE PATENTSCHRIFT

(11) **EP 0 699 091 B1**
(45) Veröffentlichungstag und Bekanntmachung des Hinweises auf die Patenterteilung: **25.11.1998**
(21) Anmeldenummer: 94915532.9
(22) Anmeldetag: 25.04.1994
(51) Int. Cl.: A61M 37/00, A61L 15/44, A61L 15/64, A61F 13/02

(54) **VERFAHREN UND VORRICHTUNG ZUR HERSTELLUNG EINES TRANSDERMALEN THERAPIESYSTEMS**
METHOD AND DEVICE FOR THE PRODUCTION OF A TRANSDERMAL THERAPEUTIC SYSTEM
PROCEDE ET DISPOSITIF POUR LA REALISATION D'UN SYSTEME THERAPEUTIQUE TRANSDERMIQUE

(30) Priorität: 19.05.1993 DE 4316751
(43) Veröffentlichungstag der Anmeldung: 06.03.1996
(73) Patentinhaber: LTS LOHMANN Therapie-Systeme GmbH, 56567 Neuwied (DE)
(72) Erfinder: BECHER, Frank, D-56072 Koblenz (DE); MÜLLER, Walter, D-56564 Neuwied (DE)
(74) Vertreter: Flaccus, Rolf-Dieter, Dr.
(86) Internationale Anmeldenummer: EP9401278
(87) Internationale Veröffentlichungsnummer: WO9426346

(56) Entgegenhaltungen:
- EP-A- 0 273 004
- DE-A- 3 642 931
- DE-A- 3 722 775

## Beschreibung

Die Erfindung betrifft ein Verfahren sowie eine Vorrichtung zur Herstellung eines transdermalen Therapiesystems zur Verabreichung von Wirkstoffen an die Haut in Form eines flächigen Mehrkammer- bzw. Mehrkammerkanalsystems.

Transdermale therapeutische Systeme mit wirkstoffgefüllten Kammern sind bekannt. Insbesondere die anfangs auf den Markt gekommenen transdermalen Systeme enthalten Kammern, die sämtlich mit Wirkstoff gefüllt sind. Die Fertigung dieser Systeme ist jedoch sehr aufwendig. Insbesondere müssen die mit Wirkstoff gefüllten "Membransäckchen" in komplizierten Arbeitsgängen einzeln hergestellt und später auf geeignete Trägerfolien im ganzen aufgebracht werden. Aus der DE-OS 37 22 775 ist ein transdermales therapeutisches System zur Verabreichung von wirkstoffen an die Haut bekannt. Es weist eine der Haut abgewandte Rückschicht, ein Wirkstoffdepot, eine wirkstoffabgabe-Steuereinrichtung, die die Abgabe des Wirkstoffes durch das System steuert, und eine haftklebende Fixierungseinrichtung für das therapeutische System an der Haut auf, wobei das Wirkstoffdepot ein Mehrkammersystem ist, in dem die diskreten Kammern einen oder mehrere Wirkstoffe aufweisen.
Eine spezielle Formgebung dieser Systeme, wie sie in der vorgenannten Schrift beschrieben ist, konnte bisher mit wirtschaftlichen Mitteln nicht verwirklicht werden. Ein weiterer Nachteil besteht darin, daR nicht genügend unterteilte Kammersysteme bei der Applikation an senkrechten Flächen dazu neigen, daß sich am unteren Rand eine Wirkstoffmasse sammelt und damit der Eintrag in die Haut an dieser Stelle besonders intensiv ist, während er im oberen Teil wesentlich geringer ist. Dies kann nur dadurch und auch nur unvollkommen vermieden werden, daß die Wirkstoffmasse sehr dickflüßig eingestellt wird, was bei der Herstellung von transdermalen Systemen Schwierigkeiten mit sich bringt und die Gestaltungsmöglichkeiten einschränkt.

Aus der DE-A-3 642 931 sind transdermale oder dermale Pflaster für die Darreichung einer kontrollierten Wirkstoffmenge bekannt, welche aus:
(1) einer geschlossenen und für die Wirkstofformulierrung undurchlässigen Deckschicht,
(2) einer für den Wirkstoff durchlässigen Schicht aus membranartigem Material, welches auf einer Seite mit der Deckschicht verbunden ist und ein Reservoir für die Wirkstoffformulierung bildet,
(3) einem Reservoir, enthaltend die Wirkstofformulierung,
(4) einer Klebschicht auf der Gegenseite der wirkstoffdurchlässigen Schicht, welche der Applikationsfläche zugewandt ist, und
(5) einer entfernbaren Schutzschicht auf der Klebschicht besteht, daß das Reservoir für die Wirkstofformulierung durch Verbindungen zwischen der Membran- und der Deckschicht in mehrere Kammern unterteilt ist.

Bei dem bekannten Mehrkammersystem kann es sich um ein solches mit unterschiedlichem Wirkstoffgehalt in den einzelnen Kammern handeln. Bei dessen Herstellung wird ein relativ aufwendiges Verfahren verwirklicht, bei welchem mehrere Portionen einer Wirkstoffsuspension nebeneinander auf die kleberfreie Seite einer Membran aufgetragen und darüber eine Deckschicht gelegt und schließlich diese an der Peripherie und zwischen den einzelnen Formulierungsportionen verschweißt wird, sodaß getrennte Wirkstoffkammern entstehen.

Der Erfindung liegt die Aufgabe zugrunde, ein sehr einfaches und damit kostengünstiges Verfahren anzugeben, mit welchem die eingangs genannten transdermalen therapeutischen Systeme auf einfache Weise und infolgedessen mit vergleichsweise hoher Produktionsgeschwindigkeit hergestellt werden. Dabei soll durch die universellen Gestaltungsmöglichkeiten der wirkstoffhaltigen Kammern oder Kanäle weitgehend unbegrenzte Freiheit bei der Entwicklung und Ausbildung neuer Systeme erreicht werden. Weiterhin soll das Verfahren besonders geeignet sein für die Verwendung flüssiger oder halbfester Wirkstoffzubereitungen, wobei insbesondere auch darauf verzichtet werden kann, den Wirkstoff mit Klebermassen zu vermischen und zu verdicken oder diesen in speziellen Materialien aufsaugen zu lassen, welche den Wirkstoff später wieder abgeben, wie dies beispielsweise bei bekannten Nitroglycerin- bzw. Nikotinpflastern gelegentlich praktiziert wird. Weiterhin besteht die Aufgabe der Erfindung in der Bereitstellung einer einfachen und insbesondere zur Durchführung eines kontinuierlichen Produktionsprozesses geeigneten Vorrichtung.

Zur Lösung der Aufgabe wird bei einem Verfahren entsprechend dem Oberbegriff von Anspruch 1 mit der Erfindung vorgeschlagen, daß die zu Verbindenden Stellen unter Verdrängung der dazwischen liegenden wirkstoffhaltigen Schicht zusammengequetscht und anschließend Ober- und Unterschicht an den Quetschstellen bleibend miteinander verbunden werden.

Mit großem Vorteil ist das vorgeschlagene Verfahren einfach und kostengünstig, es eignet sich dabei für universelle Gestaltungsmöglichkeiten der in Kammern oder Kanälen eingebetteten Wirkstoffinseln sowie zur Verwendung von flüssigen Wirkstoffen, ohne daß diese verdickt oder zusammen mit aufsaugenden Materialien vorliegen müssen.

Eine Ausgestaltung des Verfahrens sieht vor, daß die bleibende Verbindung von Ober- und Unterschicht unter Einwirkung von Druck, Hitze und/oder Ultraschall erfolgt. Eine andere Ausgestaltung sieht vor, daß die bleibenden Verbindungen durch Verkleben und/oder Verschweißen von Ober- und Unterschicht bewirkt werden.

Ein besonderer Vorteil bei der Herstellung des erfindungsgemäßen Systems liegt darin, daß die Wirkstoffmasse vollflächig aufgetragen werden kann. Im Gegensatz zu Matrixsystemen ist es nicht erforderlich, die Konsistenz der Wirkstoffschicht durch Zugabe von weiteren Stoffen relativ dickflüssig oder fest zu gestalten oder eine klebende oder mit Kleber beschichtete Oberfläche zu schaffen, welche die Kohäsion mit anderen Schichten gewährleistet. Aufwendige Auftrags- und Dosiersysteme sind bei der Herstellung des erfindungsgemäßen Systems entbehrlich.

Eine Ausgestaltung sieht vor, daß zwischen Oberschicht und Unterschicht wenigstens zwei Schichten mit unterschiedlichen Wirkstoffen eingebracht und diese Wirkstoffschichten in diskrete Kammern oder Kanäle mit unterschiedlichen Wirkstoffen unterteilt werden.

Es kann aber auch von der Maßnahme Gebrauch gemacht sein, daß zwischen Oberschicht und Unterschicht wenigstens zwei Schichten mit Wirkstoffen von unterschiedlichen Sättigungsgraden bzw. Konzentrationen eingebracht und diese Wirkstoffschichten in diskrete Kammern oder Kanäle mit Wirkstoffen von unterschiedlichen Sättigungsgraden bzw. Konzentrationen unterteilt werden.
Hierbei ist als besonderer Vorteil der unbegrenzten Gestaltungsmöglichkeiten nicht nur die Form der Reservoirkammern bzw. -flächen hervorzuheben, die in Kombination mit der Wirkstoffmasse einen großen Freiheitsgrad bei der Konzeption von transdermalen Systemen ermöglichen, sondern dies gilt insbesondere für die Nutzung der Möglichkeit, verschiedene Schichten von Wirkstoff aufzutragen oder auch mehrere Wirkstoffkammern getrennt durch Membranen, mit unterschiedlichen Sättigungsgraden zu erzeugen.
Als weiterer Vorteil ist hierbei zu bewerten, daß Wirkstoff auch in Form von Kapseln mittels Walzen aufgebracht werden kann, so daß auch die Möglichkeit besteht, hochtoxische Substanzen ohne Gefährdung für das Personal aufzubringen, und daß im übrigen solche Kapseln auch vollkommen lichtdicht sein können, weshalb auch lichtempfindliche Wirkstoffe ohne Probleme zu verarbeiten sind.

Eine Ausgestaltung sieht vor, daß als Unterschicht eine Bahn einer beschichteten oder noch zu beschichtenden Membranfolie und als Oberschicht eine Bahn eines Trägermaterials verwendet und ganz oder punktuell mit einem Kleber beschichtet wird.
Der Wirkstoff- ebenso wie der Kleberauftrag - kann aber auch nach einem vorgegebenen Muster mit Hilfe einer Technik aufgetragen werden, welche im wesentlichen der Tiefdrucktechnik entspricht. Die Bahnen werden aus geeignetem, dem Fachmann bekannten Material hergestellt, nämlich
1. Eine Membranbahn (Trägerbahn), nachstehend als Unterbahn bezeichnet und
2. eine Abdeckbahn bzw. Oberbahn.

Je nach Ausführungsform kann die Position als Ober- bzw. Unterbahn vertauscht werden. Die Herstellung der Membranbahn kann z.B. erfolgen, indem auf eine Stützschicht, die gleichzeitig die Abdeckfunktion übernimmt - z.B. eine Silikonfolie - eine Schicht aus einem hautverträglichen, für den Wirkstoff durchlässigen Kleber aufgetragen wird. Diese Kleberschicht wird mit einer als Membran geeigneten Bahn zusammenkaschiert. Bei dem Kleber handelt es sich um einen konventionellen Lösemittel- oder Heißschmelzkleber, der durch Erhitzen auftragsfähig gemacht wird. Bei der Verwendung von Lösemittelklebern müssen die Lösemittel anschließend durch Trocknen entfernt werden. Die Oberbahn wird z.B. aus einer konventionellen wirkstoffhaltigen Trägerfolie hergestellt, indem auf diese Trägerfolie ein Kleber aufgetragen wird, der in heißem Zustand (bevorzugter Temperaturbereich ca. 50-100°C) streichfähig ist, unterhalb dieses Temperaturbereiches aber fest wird und nicht mehr klebt. Wenngleich es vorteilhaft ist, daR beide Bahnen mit einem Haftschmelzkleber ausgerüstet sind, so kann doch die Ausrüstung nur einer Bahn, insbesondere der Oberbahn, mit dem Kleber ausreichend sein. Anstelle eines vollflächigen Kleberauftrages kann auf beiden Bahnen auch ein netz-, linien- oder punktförmiger Auftrag erfolgen, insbesondere auf der Membranbahn. Dadurch wird gewährleistet, daß der Durchfluß des Wirkstoffs - soweit erwünscht - nicht mehr als nötig negativ beeinflußt wird. Dies gilt insbesondere, wenn Mehrebenen-Systeme gefertigt werden. Die Trägerfolie der Oberbahn besteht aus üblichem, siegelfähigem Material, z.B. Polyethylen-Vinylacetat oder Polyethylen-Vinylalkohol. Bei der Herstellung wird die untere Bahn in eine Beschichtungsanlage gegeben und mit der wirkstoffhaltigen, flüssigen Masse - zweckmäßig vollflächig - beschichtet. Je nach Anforderung kann dieser Masse zur besseren Auftragsfähigkeit ein Lösungsmittel - auch Wasser - beigefügt werden, welches durch anschließende Trocknung wieder entfernt wird.

Eine Ausgestaltung sieht vor, daR die so hergestellte Unterbahn in einem weiteren Arbeitsgang mit der Oberbahn zusammenkaschiert wird. Ein fester Verbund kann dadurch hergestellt werden, daR beheizte Prägestempel eingesetzt werden, die den Reservoiren und Kanälen die angestrebte Form geben. Dies kann dadurch geschehen, daß sich innerhalb der Gesamtfläche des Stempels punktartig oder rasterförmig angeordnete Erhebungen befinden. Dieser Stempel wird auf zusammenkaschierten, noch nicht fest verbundenen Bahnen mit einem definierten Druck aufgebracht, so daß die zweckmäßig halbflüssige Wirkstoffmasse an den Auflagepunkten verdrängt wird. Unmittelbar nach Auftragen oder auch von Anfang an wird der Stempel so beheizt, daß der Heißschmelzkleber schmilzt und damit Unter- und Oberbahn verbindet. Entsprechend der Formgebung der Stempel erfolgt später ein Ausstanzen der fertigen Pflaster in üblicher Form.

Eine Ausgestaltung sieht vor, daß als Oberschicht eine Bahn aus einer konventionellen, wirkstoffhaltigen Trägerfolie verwendet und auf diese ein Kleber aufgetragen wird, der in heißem Zustand, bevorzugt in einem Temperaturbereich zwischen 50 und 100°C streichfähig ist, unterhalb dieses Temperaturbereichs aber fest wird und nicht mehr klebt.

Bei der Auswahl der Bahnmaterialien ist darauf zu achten, daß sie eine gewisse Druck- und Temperaturbeständigkeit aufweisen. Die Druckbeständigkeit hängt davon ab, welche Viskosität die Wirkstoffmasse aufweist und welcher Stempeldruck zur Herbeiführung einer festen Verbindung mit Hilfe der geheizten Stempel erforderlich ist. Die gewünschte Temperaturbeständigkeit der Folien hängt von der Temperatur ab, mit der die Stempel beheizt werden, d.h. es muß eine temperatur- bzw. druckbedingte Veränderung der Folien vermieden werden. Sie müssen andererseits die notwendige Elastizität aufweisen, die die Volumenverkleinerung durch die Verklebung zu kompensieren ermöglicht.
Bisher galten derartige Herstellungsverfahren als nicht praktikabel, weil es nicht möglich erschien, die aufgetragene Wirkstoffmasse an den zu heftenden Flächenteilen ausreichend zu verdrängen. In der Fachwelt wurde unterstellt, daß an den Klebstellen Reste von Wirkmasse verbleiben und eine Fremdwirkung ausüben, so daR eine zuverlässige Klebung oder Verschweißung nicht zu verwirklichen sei. Dieses Vorurteil hat sich als unzutreffend erwiesen. Bei Versuchen mit zwei Folien, zwischen denen gleichmäßig die Flüssigkeit Mybiol - ein gängiger Zusatzstoff für transdermale System - aufgebracht wurde, konnte ohne Probleme eine zuverlässige Verschweißung der Ober- und unterbahn nach einfachem Verdrängen durch den Druck der Zangen eines Schweißgerätes erzielt werden.
Eine Ausgestaltung sieht vor, daß zur Erhitzung des Heißschmelzklebers eine Einwirkung von Energie beispielsweise durch Mikrowellenstrahlung, durch Infrarotstrahlen, durch Mikrowellen-, Infrarot- oder Röntgenlaser oder Kombinationen solcher Strahlungen vorgenommen wird.
Diese Ausführungsform stellt einen weitgehend gleichmäßigen Übergang der zur Erwärmung führenden Energie sicher.
Dadurch wird der Effekt reduziert, daß zur Übertragung der Wärme aus dem Prägestempel auch die Abdeckfolie übermäßig erhitzt wird. Eine gleichmäßige Erwärmung des Siegelmaterials wird dadurch sichergestellt.

Eine Ausgestaltung sieht vor, daß im unmittelbaren Anschluß an die Verbindung von Oberschicht und Unterschicht mittels Erhitzung das so hergestellte Substrat einer bevorzugt schnellen Abkühlung insbesondere der Heftpunkte unterzogen wird. Auf diese Weise wird sichergestellt, daß eine schädliche Einwirkung der Temperaturerhöhung auf Wirkstoffmasse und andere Bestandteile des Pflasters verhindert wird. Eine Vorrichtung zur Herstellung eines transdermalen therapeutischen Systems zur Verabreichung von Wirkstoffen an die Haut in Form eines flächigen Mehrkammer- bzw. Mehrkammerkanalsystems, insbesondere zur Durchführung des Verfahrens nach der Erfindung, wobei diese Mittel zum Zusammenquetschen von einzelnen oder zusammenhängenden Stellen der Ober- und Unterschicht aufweist, ist dadurch gekennzeichnet, daß diese Mittel mit Auflage-Druckflächen ausgebildet sind, welche die den Wirkstoff einschließenden Kammern oder Kanäle unter Verdrängung der dazwischen liegenden Stellen der wirkstoffhaltigen Schicht freilassen und als Stempel oder Walzen ausgebildet sind.
Die Auflageflächen dieser Stempel können nach einer bevorzugten Ausführungsform abgerundet und/oder ellipsoid ausgebildet sein, um eine erwünschte Verteilung der Wirkstoffmasse im gesamten Pflaster herbeizuführen. Zweckmäßig ist eine leicht abgerundet Form der Auflageflächen, wodurch erreicht wird, daß zuerst in der Mitte der Auflagefläche ein Kontakt hergestellt wird, der sich dann über die gesamte Druckfläche verbreitet. Die Unterlage wird nach einer bevorzugten Ausführungsform elastisch ausgebildet. Auf diese Weise wird das Verdrängen der wirkstoffhaltigen, zweckmäßig halbflüssigen Masse in die entstehenden Aufnahmeräume erleichtern, mit der Folge, daß ein vollständiger Kontakt zwischen Heißkleberschicht und zu klebender Schicht hergestellt wird und der Klebevorgang erfolgreich abläuft.

Eine zweckmäßige Ausbildung sieht vor, daß die Auflage-Druckflächen der Stempel oder Walzen heizbar ausgebildet sind.

Nach einer weiteren Ausbildung ist vorgesehen, daß zwei Gruppen von Prägestempeln oder Prägewalzen in einer vorzugsweise kontinuierlichen Produktionslinie hintereinander angeordnet sind und übereinstimmende Prägestrukturen aufweisen, wobei die erste Gruppe zum Zusammenquetschen der Schichten des Substrats und die folgende Gruppe für deren Verbindung mit geheizten Auflageflächen ausgebildet ist.

Bei dieser Ausführungsform übernimmt die erste Stempelgruppe die Aufgabe, in der Bahn den Wirkstoff von den Flächen zu verdrängen, an denen die beiden Bahnen miteinander verklebt werden sollen. Der zweite Stempel, der praktisch auf die gleichen Auflagestellen gesetzt wird, führt dann die Heftung dadurch herbei, daß durch die Abdeckfolie hindurch der Schmelzkleber zum Erhitzen gebracht und damit eine Verbindung hergestellt wird. Bei dieser Ausführungsform ist die zweite Stempelgruppe beheizt
Anstelle der Prägestempel können auch Prägewalzen verwendet werden, wobei diese vorzugsweise Bohrungen in ihren Hohlräumen aufweisen können, um den erforderlichen Druckausgleich zu schaffen. Durch geeignete Gestaltung der Prägewalzen kann dabei der Wirkstoff vollständig in die Kammern verdrängt werden, so daR kein Wirkstoff als Abfall verloren geht.

Eine bevorzugte Ausführungsform der Stempel sieht vor, daß diese an den Auflageflächen aus einem energiedurchlässigen Material bestehen, wodurch ein energiedurchlässiger Spalt gebildet wird. Die Hohlräume werden im Gegensatz dazu aus einem reflektierenden Material gebildet. Werden diese Stempel auf die Unterbahn geführt, verdrängen sie zunächst den Wirkstoff unterhalb der Auflageflächen. Sie nähern sich dann einem Abschnitt, in dem kontinuierlich in einer Strahlungskammer Energiewellen wie Mikrowellen oder Infrarotstrahlen erzeugt werden bzw. heraustreten, wobei diese Strahlen auch gebündelt oder in Form von Lasern eingesetzt werden können, z.B. auch als Röntgenlaser. Passiert der energiedurchlässige Spalt des Stempels mit zusammengedrückten Bahnen die Auflagefläche, so tritt der Energiestrahl durch die durchlässige Auflagefläche und bewirkt eine kurzzeitige Erhitzung, die sofort wieder beendet ist, wenn der Abschnitt dahinter erreicht wird. Weil die Kammern ansonsten aus nicht durchlässigem Material wie Metall ausgebildet ist, bleibt die Wirkung auf den Bahnabschnitt beschränkt, der vom Spalt erfaßt wird. In einer bevorzugten Ausführungsform kann jeweils eine solche Kammer oben und unten angeordnet sein, um eine gleichmäßige Erhitzung der Heftpunkte sicherzustellen.
In einer weiteren bevorzugten Ausführungsform wird der Mikrowellenstrahl verstärkt, wodurch ein sogenannter "Maser" geschaffen wird. Dabei können zu Meßzwecken bevorzugt Strahlen aufgefangen und daß Ergebnis zu Meß- und Kontrollzwecken für eine Im-Prozeß-Kontrolle verwendet werden, um Angaben über Schichtdicke und spezifische Zusammensetzungen an den Meßpunkten zu erhalten.
Eine weitere bevorzugte Ausführungsform besteht darin, daß die Erzeugung der notwendigen Temperaturen durch Anlegen eines Ultraschalls an der Stempeleinheit (Stempelrolle oder Prägestempel) erfolgt.

Eine Ausgestaltung sieht vor, daß ein Prägestempel oder eine Prägewalze außer ihren Auflageflächen mit Stanz- oder Schneidwerkzeugen zur Konfektionierung von Bahnen in einzelne Substrate ausgebildet ist.
Dabei kann bei der Formgebung darauf Einfluß genommen werden, ob mit dem Stempeln bereits eine komplette Ausstanzung erfolgt oder nur ein Schneiden der entsprechenden Bahnen in kleinere Bahnen, die in einem weiteren Verarbeitungsschritt gestanzt werden. So können die Prägewalzen auch nur mit einem Messer oder Stempel ausgestattet werden, welches in Längsrichtung wirkt. Es können aber auch die Prägewalzen und Stempel zugleich die Aufgabe einer Stanzwalze übernehmen. Eine erfindungswesentliche Ausgestaltung der Vorrichtung besteht darin, daß Prägestempel oder Prägewalzen Mittel zum Anlegen von Unterdruck aufweisen. Dabei wirkt der Unterdruck auf die wirkstoffhaltige Schicht, mit der Folge, daß dieser in die bei der Prägung entstehenden Hohlräume gesaugt und von den Auflageflächen abgehoben wird.

Dabei kann eine mögliche Ausbildung der Vorrichtung zum Aufbringen von Unterdruck darin bestehen, daß der Prägestempel aus einem System eines feststehenden Kolbens mit einem zylinderähnlichen Schaft ausgebildet ist. Bei der Bewegung des Schachtes bildet sich ein Hohlraum und damit Unterdruck, wodurch die Folie in den Hohlraum des Schachtes eingesogen wird.
In einer weiteren bevorzugten Ausführung wird vom Unterdruck nach Beendigung der Heftung in einen Überdruck gewechselt, um damit die gehefteten Bahnen leichter von den Stempeln zu entfernen.

Durch eine Kombination der verschiedenen Ausbildungsmöglichkeiten kann mit der Vorrichtung nach der Erfindung ein schlüssiges Produktionsverfahren in Gang gesetzt werden. Beispielsweise werden die zusammengelegten Schichten bzw. Bahnen mit einer Transporteinrichtung unter den Prägestempel oder unter die Prägewalze gefördert, der Stempel bzw. die Walze führt einen Arbeitshub im Niedergang aus, saugt die Folie an, verdrängt dadurch den Wirkstoff, heftet mittels der Heizung, schneidet das Pflaster mit Hilfe der an seinen Kanten angeordneten Schneidmesser, löst sich aufgrund des Unterdrucks von der Transportbahn, hebt es an und "spuckt" es durch ruckartigen Übergang vom Unterdruck zum Überdruck auf ein weiteres Transportband, welches zwischen Oberbahn und Unterbahn angeordnet ist, die an dieser Stelle im Winkel nach oben bzw. nach unten so weggeführt werden, daß dazwischen Platz für die Transportbahn geschaffen ist.

Die vorbezeichneten Oberwalzen werden zweckmäßig über Unterwalzen geführt, so daß eine kontinuierliche Zu- und Abführung der zusammenzuheftenden Bahnen erfolgen kann. Dabei können sich sowohl an der Ober- als auch an der Unterwalze entsprechende Prägungen befinden, welche im Augenblick des Heftens aufeinandertreffen und somit sicherstellen, daß ein Prägedruck auf den Auflagestellen der Prägestempel erfolgt. Die Unterwalze kann dabei Vertiefungen für gegebenenfalls vorgesehene Schneidmesser enthalten, um ein sauberes Abscheren zu ermöglichen. Dabei kann zusätzlich Unterdruck an die obere Walze angelegt werden, so daß sich Hohlräume sowohl in der Ober- als auch Unterbahn bilden, in die hinein die Wirkstoffmasse von den Auflageflächen verdrängt wird.

Weiterhin können die Präge- und Verdrängungsstempel auf der Unterseite mit einem entsprechenden Stempelbett, welches ebenfalls Ausprägungen hat und in einer bevorzugten Ausführungsfrom ebenfalls einen Unterdruck aufweist, kombiniert werden.

Auch können die Prägestempel auf einem umlaufenden Band, einer Gliederkette usw. in der Art einer Raupe bei einem Geländefahrzeug angeordnet sein. Diese werden beispielsweise durch Rollen oder ähnliche Einrichtungen definiert angepreßt.

Eine weitere Ausgestaltung der Vorrichtung sieht vor, daß eine gleiche Vorrichtung auf der Unterseite angebracht ist, ggf. mit entsprechendem Gegenstempel und fallweise auch mit Unterdruck versehen.

Als Alternative zu einem beweglichen Prägestempel bzw. einer entsprechenden Walze oder mit Stempel versehenem Umlaufband, Umlaufkette o.ä. kann ein feststehendes "Prägebett" - wie bei der Fertigung von Linolschnitten - dienen. Diese Prägebetten können mit einer Bohrung zum Anlegen eines Unterdrucks versehen werden. Im ersten Schritt wird die Bahn auf das Prägebett geführt, es wird ein Unterdruck angelegt, so daß sich Hohlräume bilden, gleichzeitig fährt ein flacher Stempel auf dieses Bett und verdrängt an den Auflageflächen des Prägebettes die Wirkstoffzubereitung und sorgt damit für eine Befüllung der Kammern. Das "Prägebett" kann ebenfalls auf einem Umlaufband befestigt werden; auch der Flachstempel kann durch eine Walze oder ein entsprechendes Umlaufband ersetzt werden.

Die Vorrichtung kann auch so ausgebildet sein, daß die Stempel nicht im rechten Winkel zu der zu prägenden Bahn geführt werden, sondern auf einer Walze aufgebracht werden, im Prinzip ähnlich wie die Walzen, mit denen im Haushalt Backwaren geformt werden (Model-Walzen). Diese Ausführungsform hat den besonderen Vorteil, daß die Wirkstoffmasse nicht nur in die entstehenden Reservoire gedrängt wird, sondern quasi als "Welle" vor dem Auflagepunkt der Walze geführt und damit sinnvoll eingesetzt wird. Damit wird die Entstehung von Spannungen im Material reduziert.

Eine andere Ausführung besteht darin, daß zwei Walzen - analog den zwei Stempelgruppen - eingesetzt werden, die die Formgebung beeinflussen. Dabei hat die erste Walze die Aufgabe, den Wirkstoff von den Auflagepunkten zu verdrängen, und die zweite Walze die Aufgabe, die fest haftende Verbindung durch Erhitzen herbeizuführen.

Ein bevorzugte Ausführungsform des Prägestempels bzw. der Walze besteht darin, daß dieselbe in den Aussparungen hohl ausgebildet wird und daß an diese Aussparung in dem Moment ein Unterdruck angelegt wird, in dem der Prägestempel auf die Oberfolie trifft. Dadurch wird die Oberfolie in definiertem Maße in die Aussparungen des Prägestempels gezogen und vorgespannt. Dies führt zu einem Unterdruck zwischen Ober- und Unterfolie mit der Wirkung, daR der Wirkstoff von den Auflageflächen hin in die jeweilige Mitte von Kammer und Kanälen gezogen wird, so daß der Verdrängungseffekt an den Auflageflächen unterstützt wird.

Weiterhin kann von der Maßnahme Gebrauch gemacht sein, daß die Abdeckfolie vor dem Zusammenführen mit der Wirkstoffolie prozeßähnlich mit einer - allerdings nur auf eine geringe Tiefe wirkenden - Tiefziehfolie ausgestattet wird, indem sie über die Positiv-Negativ-Stempel einer Prägeeinrichtung mit einer Siegelfolie zusammenkaschiert wird. Dies kann bevorzugt in einer dem Tiefdruckverfahren ähnlichen wie folgt erfolgen:

Die mit Aussparungen versehenen Stempel bzw. Walzenstempel laufen in einer beheizten Walze. Sie werden in der Aussparung mit Heißkleber befüllt, die erhabenen Partien werden mit Hilfe eines Rakels gesäubert; anschließend laufen Walze und Folie (Oberbahn) zusammen und der Kleber wird auf die Folie an die genau definierten Heftflächen abgegeben. Zur Vermeidung der Verklebung des Heißklebers mit Stempel bzw. Stempelwalze werden diese entsprechend beschichtet, bevorzugt mit Silikonzubereitungen oder Polytetrafluorethylen. Dies kann auch erfolgen, nachdem sie bereits mit einem Schmelzkleber beschichtet worden sind.

Es ist aber auch möglich, daß der Verarbeitungsschritt vor dem Auftrag der Wirkstoffmasse durch eine beheizte Walze erfolgt, wobei dann vorteilhaft nur die erhabenen Auflagepunkte mit Schmelzkleber beschichtet werden. Anstelle eines Heißschmelzklebers kann auch ein geeignetes Siegelmaterial eingesetzt werden, das bei Zuführung durch Hitze klebende Eigenschaften erhält und diese klebenden Eigenschaften bei der Rückkehr zu normalen Temperaturverhältnissen verliert.

Eine bevorzugte Arbeitsweise sieht vor, daß in die geprägten Reservoirräume nach Art eines Tiefdruckverfahrens oder eines sonstigen geeigneten Druckverfahrens der Wirkstoff aufgetragen wird. Dies kann über die Hohlräume eines anderen Walzensystems - wie beim Tiefdruck - erfolgen. In einem anschließenden Verarbeitungsschritt würde dann im Anschluß an den Wirkstoffauftrag, der in diesem Falle nicht vollflächig erfolgt - die fest haftende Verbindung nach den oben beschriebenen Prinzipien erfolgen.

Die Arbeitsweise kann dahingehend modifiziert werden, daß der Wirkstoffauftrag wie oben beschrieben erfolgt, jedoch nicht in vorgeprägte Hohlräume, sondern flächig. Da die Menge der Wirkstoffmasse gering ist, bedarf es einer Ausprägung in vielen Fällen nicht. Die Elastizität der Folie reicht dann aus, um die erwünschten Hohlräume nach der Verfestigung über den Heißklebeprozeß zu gewährleisten.

Bei einem vollflächigen Auftrag übt der Stempel keine Verdrängungsfunktion aus und es sind apparativ lediglich jeweils ein Stempel bzw. die Stempelwalze (die gleichzeitig Schnittwalze sein kann) erforderlich.
Es kann zweckmäßig sein, die Beschichtung mit dem Heißkleber auf dem Auflagepunkt ebenfalls nach Art eines Tiefdruckverfahrens über eine entsprechende Walze auf den späteren Klebepunkten vorzunehmen und anschließend den Wirkstoff aufzutragen. Auch eine umgekehrte Reihenfolge ist möglich.

Wenn auf die Beschichtung der Trägerfolie mit Heißkleber verzichtet wird, können statt dessen kleine Heißkleberstücke, z.B. in Kugelform, mit der Wirkstoffmasse vermischt im Rahmen der Masseaufbereitung und zusammen mit dieser aufgetragen werden.

Wenn der Prägestempel (ggf. auch Walze oder Umlaufband) sich von oben der Unterlage nähert, wird zunächst ein Teil der Masse mit den Kügelchen verdrängt; da diese aber fest sind, verbleiben etliche von ihnen in der Auflagefläche. Dadurch entsteht auf den - kleinen - Auflageflächen ein erhöhter spezifischer Druck, der die Verdrängung der Wirkstoffmasse verbessert; diese kann in den Zwischenräumen besonders gut abfließen. Durch die Erhitzung mit Hilfe des Heftstempels werden die Schmelzkleberteilchen pastös bzw. flüssig und verbinden sich mit Ober- und Unterfolie, so daß eine Haftung entsteht. Bevorzugt können auch (mikro)verkapselte konventionelle Kleber verwendet werden, die der Wirkstoffmasse beigemischt und wie zuvor aufgetragen werden. In diesem Falle bedarf es zur Haftung keines beheizten Stempels. Allein durch den Druck des Stempels platzen die Kapseln, nachdem zuvor aufgrund der relativ größeren "Härte" der Kapseln ein Großteil der Wirkstoffmasse stärker verdrängt worden ist.

Eine Abwandlung des Arbeitsverfahrens sieht vor, daß eine Masse bestehend aus konventionellen Klebern und mikroverkapselten weiteren Kleberschichten mit Hilfe eines Tiefdruckverfahrens an den späteren Fixierpositionen der Unterbahn (Trägerbahn) aufgetragen wird. Soweit die Kleber Lösemittel enthalten, werden sie auf konventionelle Weise getrocknet. Unter der Trägerfolie kleben somit auf den späteren Heftflächen relativ feste Klebstoffteilchen. Beim Prägevorgang sorgen diese dafür, daß zunächst der Wirkstoff verdrängt wird. In einer Endphase platzen die Kapseln und führen die Heftung herbei. Diese Methode läßt sich somit auch für temperaturempfindliche Wirkstoffmassen anwenden. Der Vorteil gegenüber einer einfachen Beschichtung liegt darin, daß die Wirkstoffmasse sich nicht mit dem Kleber verbindet und somit nicht eine Zwischenschicht schafft, die eine Verbindung mit der unteren Membranfolie verhindert. In einem oder mehreren Arbeitsgängen nach den vorgenannten Verfahren können auf die definierte Fläche an den späteren Heftpunkten mit Hilfe von geeignetem schnelltrocknenden Lösungsmittelkleber kleine, verkapselte konventionelle Kleberteilchen aufgebracht werden. Hier kann die Fixierung also allein durch den entsprechenden Druck erfolgen. Durch diesen Druck wird zunächst die gegebenenfalls darunter befindliche Wirkstoffmasse verdrängt; in einer weiteren Phase platzen die Mikrokapseln und geben den Kleber frei, der dann zu einer Verfestigung zwischen Ober- und Unterbahn führt.

Ein besonderer Vorteil der Erfindung liegt auch darin, daß sich Herstellung und Entwicklung weitgehend standardisieren und sich die meisten Komponenten auf wenige Typen reduzieren und vorfertigen lassen.

Zum Beispiel dürfte es genügen, wenige Membrantypen unterschiedlicher Durchlässigkeit zu beschichten, dabei brauchen nur wenige verschiedene Kleber für die jeweilige Membran ausgewählt zu werden.

Noch günstiger liegen die Verhältnisse bei der Oberbahn, die ja für die unterschiedlichsten transdermalen Therapiesysteme gleich sein kann, da sie nur dicht und verträglich mit der Wirkstoffmasse sein muß. Die Anforderungen an diese Komponenten sind so gering, daß eine GMP-gerechte Fertigung dieser Komponenten nicht notwendig ist. Auch die Stempel lassen sich universell einsetzen.

Gegenüber den bekannten Beutelpflastern hat die Erfindung den Vorteil, daß keine "Ausbauchung" an senkrechten Flächen eintritt und eine gleichmäßige Konzentration auch im oberen Bereich vorhanden ist. Das Verfahren ist daher insbesondere dann geeignet, wenn flüssige oder halbflüssige Wirkstoffzubereitungen eingesetzt werden sollen, deren Verwendung im Zusammenhang mit Klebemassen problematisch ist, z.B. wegen schlechter Mischbarkeit, Verlust der Klebkraft usw. Es eignet sich besonders gut für Fälle, in denen eine schnelle Diffusion durch die Membran - ungehindert vom Kleber - gewünscht wird, weil dann gleichwohl eine sichere Befestigung dadurch erreicht wird, daß die Flächen unterhalb der "Kanäle" vom Wirkstoff freigehalten werden und durch die Klebflächen zwischen den "Kanälen" festgehalten werden.
Ein weiterer Vorteil der Erfindung besteht in der Möglichkeit, halbflüssige Massen zu verwenden, deren Wirkstoff eine stärkere Mazerationswirkung hat. Dies kann vermindert werden dadurch, daß die den Wirkstoff enthaltenden Kammern so gestaltet werden, daß die eigentlichen Klebepunkte an den Heftstellen wirkstofffrei gestaltet werden. Es kann so quasi ein "Raster" oder ein "Gitter" an Auflagefläche geschaffen werden, auf welchem der Wirkstoff auf die Haut wirkt. Dies erfordert zwar eine etwas größere Fläche des Pflasters, wird aber durch die Vorteile überkompensiert.

Besonders vorteilhaft erweist sich das erfindungsgemäße Verfahren wenn die Wirkstoffmasse dazu neigt, mit dem Kleber unter bestimmten Temperaturbedingungen zu reagieren ("tempern"). So ist z.B. bekannt, daß das Selegilin-Hydrochlorid mit den basischen Komponenten eines Acrylklebers reagiert, so daß Selegilin-Base entsteht, die nicht die für die transdermale Anwendung gewünschten Eigenschaften hat.

Verwendet man ein Multikanalsystem, so kann auf Acrylkleber völlig verzichtet werden, und der Hautkontakt der Wirkstoffe kann völlig ohne Kleber erreicht werden.

Sehr vorteilhaft wirkt sich das Verfahren auch bei der Entwicklung von "Screening" der Hautgängigkeit von Wirkstoffen aus, dadurch werden bei der Entwicklung von transdermalen Systemen erste Erkenntnisse gewonnen.
Es können dabei verschiedene Wirkstoffmassen schnell mit vorgefertigten Komponenten kombiniert und entsprechende Pflaster erfindungsgemäß z.B. über einen einfachen Auftragskasten mit einer beheizten Walze hergestellt werden. Eine umständliche Ermittlung geeigneter Mischungen von Klebern mit Wirkstoffmassen entfällt, gleichermaßen die Suche nach einem geeigneten Absorbierungsmaterial wie Vliesstoff.

### Beispiele:

1. Auf eine Unterbahn (Membran) wird flächig eine Masse mit einer Wirkstoffzubereitung aufgetragen. Die Unterbahn und eine durch Beschichten einer Abdeckfolie mit einem hautverträglichen, wirkstoffdurchgängigen Heißschmelzkleber und Zukaschieren einer Membranfolie, die den Wirkstoff kontrolliert durchläßt, erhaltene Oberbahn werden zusammenkaschiert und der Heftstation zugeführt. Diese besteht aus einem ersten Speicherplatz, einem Zuführungsplatz, einem Heftplatz, einem Wegführungsplatz, einem zweiten Speicherplatz und einem Weitertransportplatz. Die zusammenkaschierte Bahn die wegen der Zwischenschicht aus Wirkstoffmasse nicht fest verbunden ist wird dem Speicherplatz zugeführt. Hinter dem Speicherplatz wird sie von der Klemmeinrichtung des Zuführungsplatzes und gleichzeitig vom Wegführungsplatz festgehalten und gespannt. Hinter dem Zuführungsplatz befindet sich der Heftplatz. Hier wird zunächst ein Verdrängungsstempel auf die Bahnen geführt, der den Wirkstoff an den Präge-Auflageflächen verdrängt. Durch eine Transportbewegung der Gesamttransporteinrichtung wird die Folie weitergeführt zum eigentlichen Heftplatz. Hier wird ein geheizter Prägestempel präzis auf die gleichen Stellen geführt, bei denen der vorgehende Verdrängungsstempel den Wirkstoff zwischen den Folien verdrängt hat. Der Heizstempel hat in Abhängigkeit von der Adhäsion, Kohäsion und Viskosität der Wirkstoffmasse eine etwas kleinere Auflagefläche als der Verdrängungsstempel, so daß ein etwaiger Rückfluß der Wirkstoffmasse in dem zwischenzeitlichen Transportschritt in die alte Lage weitgehend verhindert wird.
   Durch den Heizstempel wird das System an verschiedenen Punkten dauerhaft fixiert, indem Schmelzkleber oder Siegelfolie zwischen Trägerfolie und Unterbahn zum Kleben gebracht wird. In einem weiteren Transportschritt wird die Folie mit den strukturierten Systemen zum Speicher und dann der Transportwalze weiterbefördert und später vereinzelt verpackt.
2. Unterbahn und Oberbahn werden gemäß Beispiel 1 zusammenkaschiert und der Heftstation zugeführt. Abweichend vom Beispiel 1 werden aber der Verdrängungsvorgang und der Heftvorgang in einem Schritt vorgenommen, d.h. der Heftstempel ist beheizt und verdrängt in einem Arbeitsschritt die Wirkstoffmasse zwischen den beiden Bahnen und hält die Heißkleberschicht auf, so daß sich Ober- und Unterbahnen an den Auflageflächen verbinden.
   Dabei kann der Verdrängungs- und Heftschritt in Teilschritten so erfolgen, daß der Stempel zunächst so geführt wird, daß lediglich eine Verdrängung stattfindet und erst nach kurzem Zeitablauf - nach dem eingetretenen Erfolg - ein weiterer Druck ausgeübt wird, so daß die Haftung unter Druck erfolgt. Dabei kann der zweite Teilschritt auch so erfolgen, daß über einen rotierenden Noppen die Unterbahn gegen den Stempel geführt wird. Diese Variante hat den Vorzug, daß etwaige Spannungen der Folie sich gleichmäßig auf Unterbahn und Oberbahn verteilen, so daß sich die fertigen Systeme nicht aufgrund unterschiedlicher Spannungsverhältnisse krummen.
3. Man verfährt wie nach Beispiel 1 bzw. 2 mit der Maßgabe, daß der Heftstempel auch dazu genutzt wird, die Pflaster zu vereinzeln, indem die Ränder scharfkantig in Art einer Stanze ausgebildet werden; die Klingen können so ausgebildet werden, daß sie durch Druck in entsprechende Schlitze auf der Auflage eingreifen, so daß ein sauberer Schnitt erreicht wird.
4. Man verfährt wie nach Beispiel 2. Anstelle eines Verdrängungsstempels und eines Heftstempels bzw. eines Kombinationsstempels, evtl. kombiniert mit den Stanzklingen gemäß Beispiel 3, werden rotierende Stempelwalzen - in der Art von Rollmodeln für das Backen - eingesetzt. Dies hat den Vorteil, daß Speicherplatz und eine Zuführungseinrichtung entbehrlich werden- die obere Stempelwalze kann beheizt werden. Eine vorteilhafte Abwandlung kann man dann erreichen, wenn Verdrängung und/oder Heftung durch die Kombination einer Oberwalze mit einer zweckmäßig ausgebildeten Unterwalze erfolgen. Dabei kann die Unterwalze so ausgebildet werden, daß sie noppenförmige Erhöhungen an geeigneten Stellen aufweist, um Verdrängung und Heftung in zwei verschiedenen Abschnitten zu fördern. Ein getrenntes Zuführungsteil ist in diesen Fall entbehrlich oder kann einfacher ausgebildet werden. Die Verdrängungs-/Heftungswalze kann als Schneidwalze ausgeführt werden, ebenfalls in Kombination mit einer entsprechenden Unterwalze. Zusätzlich können Abnahmevorrichtungen für die fertig gestanzten Pflaster vorgesehen werden.
5. Man stellt eine Oberbahn und eine Unterbahn her und verbindet diese, wie in den Beispielen 1 und 2 beschrieben, jedoch mit der Abweichung, daß die Oberbahn nicht vollflächig mit Schmelzkleber beschichtet wird, sondern in der Art eines Druckverfahrens durch einen beheizten Tiefdruckzylinder, der in einer beheizten Wanne rotiert. In dem Zylinder sind geringfügige Vertiefungen, die mit Hilfe eines Rakels abgestrichen werden. Dann wird der Schmelzkleber auf die Bahn geführt und bleibt dort kleben. Bei diesem Verfahren können die gewünschten Muster für Schmelzklebepunkte und Aussparungen für die pastöse Wirkstoffmasse vorgesehen werden.
   Der Auftrag der Wirkstoffmasse kann in entsprechend komplimentären Mustern wahlweise auf die obere Bahn - von unten - erfolgen. in diesem Fall wird die Wirkstoffmasse so eingestellt, daß die Viskosität und Adhäsion, Kohäsion und Klebrigkeit ausreicht, um den Wirkstoff bis zur Heftung auf bzw. unter der Folie festzuhalten. Die übrigen Schritte der Heftung erfolgen wie oben angegeben. Das Gleiche gilt für die Vereinzelung.
6. Man stellt ebenfalls zwei Bahnen her. Die - bisher oben befindliche Trägerfolie wird jedoch nach unten gesetzt. An den erwünschten Fixierpunkten wird zunächst in dem Tiefdruckverfahren ähnelndem Prozeß Heißkleber definiert auf die Fixierpunkte aufgetragen. Dieser Prozeß erfolgt bei gewendeter Folie, d.h. auf der Seite, die später statt unten unter der Folie oben auf der Folie liegt. Der Auftrag des Heißklebers erfolgt - evtl. in mehreren Schritten - in einer Dicke, daß sich zwischen den Kleberpunkten erfindungsgemäß vorgesehene Kanäle und Kammern bilden. Diese Kammern werden dann vollflächig mit Wirkstoff beschichtet- an den Auflagepunkten des Heißklebers werden überschüssige Wirkstoffmengen mit Hilfe eines Rakels abgestrichen.
   Der Prozeß der Verfestigung erfolgt nach einem Verfahren gemäß den o.a. Beispielen mit der Maßgabe, daß nunmehr nur ein Heftschritt erforderlich ist, nicht aber ein Verdrängungsschritt.
7. Die Herstellung erfolgt gemäß Beispiel 1 bis 6, jedoch wird eine Trägerfolie verwendet, die sich zum Tiefziehen eignet und somit über einen Prägestempel oder entsprechende Prägewalzen die erwünschten Vertiefungen bzw. Erhöhungen erhält. Diese Vertiefungen und Erhöhungen werden gemäß Beispiel 1 bis 6 mit Heißkleber bzw. Mikrokapselkleber beschichtet und mit Wirkstoffmasse gefüllt, geheftet und gegebenenfalls vereinzelt abgenommen.

## Patentansprüche

1. Verfahren zur Herstellung eines transdermalen Therapiesystems zur Verabreichung von Wirkstoffen an die Haut in Form eines flächigen Mehrkammer- bzw. Mehrkammerkanalsystems, bei welchem eine geeignete Oberschicht und eine Unterschicht bereitgestellt werden, zwischen diesen Schichten mindestens eine wirkstoffhaltige Schicht eingebracht und anschließend die Ober- und Unterschicht an vereinzelten und/oder zusammenhängenden Stellen unter Ausbildung von wirkstoffhaltigen Kammern oder Kanälen miteinander verbunden werden, dadurch gekennzeichnet, daß die zu verbindenden Stellen unter Verdrängung der dazwischen befindlichen wirkstoffhaltigen Schicht zusammengequetscht und anschließend Ober- und Unterschicht an den Quetschstellen bleibend miteinander verbunden werden.

2. Verfahren nach Anspruch 1, dadurch gekennzeichnet, daß die bleibende Verbindung von Ober- und Unterschicht unter Einwirkung von Druck, Hitze und/oder Ultraschall erfolgt.

3. Verfahren nach Anspruch 1 oder 2, dadurch gekennzeichnet, daß die bleibenden Verbindungen durch Verkleben und/oder Verschweißen von Ober- und Unterschicht bewirkt werden.

4. Verfahren nach Anspruch 1, dadurch gekennzeichnet, daß zwischen Ober- und Unterschicht wenigstens zwei Schichten mit unterschiedlichen Wirkstoffen eingebracht und diese Wirkstoffschichten in diskrete Kammern oder Kanäle mit unterschiedlichen Wirkstoffen unterteilt werden.

5. Verfahren nach Anspruch 1, dadurch gekennzeichnet, daß zwischen Oberschicht und Unterschicht wenigstens zwei Schichten mit Wirkstoffen von unterschiedlichen Sättigungsgraden bzw. Konzentrationen eingebracht und diese Wirkstoffschichten in diskrete Kammern oder Kanäle mit Wirkstoffen von unterschiedlichen Sättigungsraten bzw. Konzentrationen unterteilt werden.

6. Verfahren nach einem oder mehreren der Ansprüche 1 bis 5, dadurch gekennzeichnet, daß das Zusammenquetschen und Verbinden von Oberschicht und Unterschicht gemeinsam in einem Arbeitsschritt oder nacheinander in zwei getrennten Arbeitsschritten durchgeführt wird.

7. Verfahren nach einem der Ansprüche 1 bis 6, dadurch gekennzeichnet, daß als Unterschicht eine Bahn einer beschichteten oder noch zu beschichtenden Membranfolie und als Oberschicht eine Bahn eines Trägermaterials verwendet und ganz oder punktuell mit einem Kleber beschichtet wird.

8. Verfahren nach Anspruch 7, dadurch gekennzeichnet, daß der Wirkstoff- ebenso wie der Kleber nach einem vorgegebenen Muster mit Hilfe einer Technik aufgetragen werden, welche im wesentlichen der Tiefdrucktechnik entspricht.

9. Verfahren nach einem oder mehreren der Ansprüche 1 bis 8, dadurch gekennzeichnet, daß die unterschiedlichen Schichten in Form von Bahnen im Laufe eines kontinuierlichen Fertigungsprozesses zusammengeführt und unter Verwendung einer Quetschvorrichtung mit Mitteln zum streckenweisen Zusammenquetschen unter Freilassung von wirkstoffgefüllten Kammern oder Kanälen fortlaufend zusammengequetscht und miteinander verbunden werden.

10. Verfahren nach einem oder mehreren der Ansprüche 1 bis 9, dadurch gekennzeichnet, daß die Formgebung der Kammern oder Kanäle durch beheizte Prägestempel oder Prägewalzen unter Verwendung von Heißklebern oder durch Druck unter Verwendung von druckempfindlichen Klebern mit ungeheizten prägestempeln bzw. Prägewalzen erfolgt.

11. Verfahren nach einem oder mehreren der Ansprüche 1 bis 10, dadurch gekennzeichnet, daß als Oberschicht eine Bahn aus einer konventionellen, wirkstoffhaltigen Trägerfolie verwendet und auf diese ein Kleber aufgetragen wird, der in heißem Zustand, bevorzugt in einem Temperaturbereich zwischen 50 und 100°C streichfähig ist, unterhalb dieses Temperaturbereiches aber fest wird und nicht mehr klebt.

12. Verfahren nach Anspruch 11, dadurch gekennzeichnet, daß sowohl die Oberschicht als auch die Unterschicht bzw. die entsprechenden Bahnen mit einem Heißschmelzkleber ausgerüstet sind.

13. Verfahren nach einem der Ansprüche 1 bis 12, dadurch gekennzeichnet, daß anstelle eines vollflächigen Kleberauftrages bei einer oder beiden Schichten bzw. Bahnen ein netz-, linien- oder punktförmiger Auftrag erfolgt, insbesondere auf der unteren Membranschicht.

14. Verfahren nach einem oder mehreren der Ansprüche 1 bis 13, dadurch gekennzeichnet, daß zur Erhitzung des Heißschmelzklebers eine Einwirkung von Energie beispielsweise durch Mikrowellenscrahlung, durch Infrarotstrahlen, durch Mikrowellen-, Infrarot- oder Röntgenlaser oder Kombinationen solcher Strahlungen vorgenommen wird.

15. Verfahren nach einem oder mehreren der Ansprüche 1 bis 14, dadurch gekennzeichnet, daß in unmittelbarem Anschluß an die Verbindung von Oberschicht und Unterschicht mittels Erhitzung das so hergestellte Substrat einer bevorzugt schnellen Abkühlung insbesondere der Heftpunkte unterzogen wird.

16. Vorrichtung zur Herstellung eines transdermalen therapeutischen flächigen Mehrkammer- bzw. Mehrkammerkanalsystems zur Verabreichung von Wirkstoffen an die Haut flächigen Mehrkammer- bzw. Mehrkammerkanalsystems, insbesondere zur Durchführung des Verfahrens nach der Erfindung, wobei die Vorrichtung Mittel zum Zusammenquetschen von einzelnen oder zusammenhängenden Stellen der Oberschicht und Unterschicht aufweist, dadurch gekennzeichnet, daß diese Mittel mit Auflagedruckflächen ausgebildet sind, welche den Flächen zwischen den wirkstoffeinschließenden Kammern oder Kanälen entsprechen und als Stempel oder Walzen ausgebildet sind.

17. Vorrichtung nach Anspruch 16, dadurch gekennzeichnet, daß die Auflage-Druckflächen der Stempel oder Walzen heizbar ausgebildet sind.

18. Vorrichtung nach Anspruch 17, dadurch gekennzeichnet, daß einem Prägestempel oder einer Prägewalze eine Unterlage zugeordnet und diese vorzugsweise elastisch ausgebildet ist.

19. Vorrichtung nach einem der Ansprüche 1 bis 18, dadurch gekennzeichnet, daß zwei Gruppen von Prägestempeln oder Prägewalzen in einer vorzugsweise kontinuierlichen Produktionslinie hintereinander angeordnet sind und übereinstimmende Prägestrukturen aufweisen, wobei die erste Gruppe zum Zusammenquetschen der Schichten des Substrats und die folgende Gruppe für deren Verbindung mit geheizten Auflageflächen ausgebildet ist.

20. Vorrichtung nach einem der Ansprüche 1 bis 19, dadurch gekennzeichnet, daß in der Produktionslinie eine Kühlstation, beispielsweise mit einem Heißluftgebläse zur vorzugsweise schnellen Kühlung von Heißverbindungsstellen angeordnet ist.

21. Vorrichtung nach einem oder mehreren der Ansprüche 1 bis 20, dadurch gekennzeichnet, daß ein Prägestempel oder eine Prägewalze außer ihren Auflageflächen mit Stanz- oder Schneidwerkzeugen zur Konfektionierung von Bahnen in einzelne Substrate ausgebildet ist.

22. Vorrichtung nach einem oder mehreren der Ansprüche 1 bis 21, dadurch gekennzeichnet, daß diese Mittel zum Anlegen von Unterdruck an einen Prägestempel oder eine Stempelwalze aufweist.

23. Vorrichtung nach einem oder mehreren der Ansprüche 1 bis 22, dadurch gekennzeichnet, daß jeweils zwei Prägestempel oder Prägewalzen von oben oder unten gegeneinander bewegbar angeordnet und unter Druckeinrichtung miteinander zusammenwirkend ausgebildet sind.

## Claims

1. A process for the manufacture of a transdermal therapeutic system for the administration of active substances to the skin in the form of a sheet-like multichamber or multichamber-channel system, wherein a suitable top layer and a bottom layer are provided, at least one active substance-containing layer is introduced between these layers, and subsequently top and bottom layer are bonded with each other at individual and/or coherent sites under formation of active substance-containing chambers or channels, characterized in that the sites to be bonded are squeezed under displacement of the intermediary active substance-containing layer, and that subsequently top and bottom layer are permanently bonded with each other at the squeezed sites.

2. The process according to claim 1 characterized in that the permanent bond of upper and lower layer is effected under the action of pressure, heat, and/or ultrasonics.

3. The process according to claim 1 or 2 characterized in that the permanent bonds are effected by gluing and/or welding the upper and lower layer.

4. The process according to claim 1 characterized in that at least two layers having different active substances are introduced between upper and lower layer, and that these active substance layers are subdivided into discrete chambers or channels having different active substances.

5. The process according to claim 1 characterized in that at least two layers having active substances of different saturation degrees or concentrations are introduced between top layer and bottom layer, and that these active substance layers are subdivided into discrete chambers or channels having active substances of different saturation degrees or concentrations.

6. The process according to one or several of claims 1 to 5 characterized in that squeezing and bonding of upper layer and lower layer is carried out together in one operation or in succession in two separate operations.

7. The process according to any one of claims 1 to 6 characterized in that a web of a coated membrane sheet or one still to be coated is used as bottom layer, and a web of a carrier material is used as top layer, and that an adhesive is applied completely or selectively.

8. The process according to claim 7 characterized in that the active substance as well as the adhesive is applied according to a given pattern by means of a technique substantially corresponding to gravure printing.

9. The process according to one or several of claims 1 to 8 characterized in that the different layers in the form of webs are joined within the course of a continuous production process and are continuously squeezed and bonded together by using a squeezing device having means for squeezing in parts, under leaving open chambers or channels filled with active substance.

10. The process according to one or several of claims 1 to 9 characterized in that forming of the chambers or channels is carried out by means of heated embossing punches or embossing rolls using hot-melt adhesives, or by means of pressure using pressure-sensitive adhesives with unheated embossing punches or embossing rolls.

11. The process according to one or several of claims 1 to 10 characterized in that a web of a conventional, active substance-containing supporting sheet is used as top layer, and that an adhesive is applied thereon which is spreadable in hot condition, preferably in a temperature range between 50 and 100°C, but becomes solid below said temperature range and ceases to be tacky.

12. The process according to claim 11 characterized in that both top layer and bottom layer or the corresponding webs are provided with a hot-melt adhesive.

13. The process according to any one of claims 1 to 12 characterized in that instead of an all-over spread, a spread in the form of a net, lines, or spots is applied on one or both layers or webs, in particular on the lower membrane sheet.

14. The process according to one or several of claims 1 to 13 characterized in that heating the hot-melt adhesive is effected by an action of energy, for example, by means of microwave radiation, infrared radiation, by means of microwave, infrared, or x-ray lasers, or by combinations of such radiation.

15. The process according to one or several of claims 1 to 14 characterized in that immediately after bonding upper layer and lower layer by means of heating the substrate thus produced is subjected to cooling, preferably rapid cooling, in particular of the bonding points.

16. A device for producing a transdermal therapeutic plane multichamber or multichamber-channel system for the administration of active substances to the skin a plane multichamber or multichamber-channel system, in particular for carrying out the process according to the present invention, wherein said device has means for squeezing individual or coherent points of top layer and bottom layer, characterized in that said means are provided with contact-bearing-surfaces corresponding to the surfaces between the chambers or channels enclosing the active substance, and that they are formed as punches or rolls.

17. The device according to claim 16 characterized in that said contact-bearing-surfaces of the punches or rolls may be heated.

18. The device according to claim 17 characterized in that a support is assigned to one embossing punch or one embossing roll, and that this preferably is flexible.

19. The device according to any one of claims 1 to 18 characterized in that two groups of punches or embossing rolls are arranged in series in a production line, which preferably operates in continuous mode, and that these have corresponding embossing structures, the first group being formed to squeeze the layers of the substrate, and the following group being provided with heated contact surfaces to bond these layers.

20. The device according to any one of claims 1 to 19 characterized in that a cooling station, for example with a hot blast, is arranged in the production line to cool heat-bonded sites, preferably in a rapid manner.

21. The device according to one or several of claims 1 to 20 characterized in that one embossing punch or one embossing roll, in addition to their contact surfaces, is provided with punching or cutting tools to manufacture webs into individual substrates.

22. The device according to one or several of claims 1 to 21 characterized in that it has means for applying a vacuum to an embossing punch or an punching roll.

23. The device according to one or several of claims 1 to 22 characterized in that two embossing punches or embossing rolls are each arranged from above or below in a mutually movable manner, and that they are formed to cooperate under pressure.

## Revendications

1. Procédé de fabrication d'un système thérapeutique transdermique pour l'administration de principes actifs à la peau sous forme d'un système plat à chambres multiples resp. à canaux à chambres multiples, consistant à prévoir une couche supérieure appropriée et une couche inférieure, à insérer entre ces couches au moins une couche contenant des principes actifs et à relier ensuite entre elles les couches supérieure et inférieure à des endroits séparés et/ou contigus en formant des chambres ou canaux contenant des principes actifs, caractérisé en ce que les endroits à relier sont comprimés en supprimant la couche intercalée contenant des principes actifs et aux endroits de compression, les couches supérieure et inférieure sont ensuite reliées entre elles de façon permanente.

2. Procédé selon la revendication 1, caractérisé en ce que la liaison permanente entre les couches supérieure et inférieure est obtenue sous l'effet de pression, chaleur et/ou ultrason.

3. Procédé selon la revendication 1 ou 2, caractérisé en ce que les liaisons permanentes sont obtenues par collage et/ou soudage des couches supérieure et inférieure.

4. Procédé selon la revendication 1, caractérisé en ce qu'au moins deux couches contenant des principes actifs différents sont insérées entre les couches supérieure et inférieure et ces couches de principes actifs sont divisées en chambres ou canaux discrets contenant des principes actifs différents.

5. Procédé selon la revendication 1, caractérisé en ce qu'au moins deux couches contenant des principes actifs de degrés de saturation resp. de concentrations différentes sont insérées entre la couche supérieure et la couche inférieure et ces couches de principes actifs sont divisées en chambres ou canaux discrets contenant des principes actifs de taux de saturation resp. de concentrations différentes.

6. Procédé selon l'une ou plusieurs des revendications 1 à 5, caractérisé en ce que la compression et la liaison de la couche supérieure et de la couche inférieure sont effectuées simultanément en une opération, ou successivement en deux opérations séparées.

7. Procédé selon l'une des revendications 1 à 6, caractérisé en ce qu'une bande d'une feuille à membrane enduite ou encore à enduire est utilisée comme couche inférieure et une bande d'un matériau support est utilisée comme couche supérieure et enduite entièrement ou partiellement d'un adhésif.

8. Procédé selon la revendication 7, caractérisé en ce que le principe actif - de même que l'agent adhésif - est appliqué selon un modèle donné, à l'aide d'une technique correspondant essentiellement à l'héliogravure.

9. Procédé selon l'une ou plusieurs des revendications 1 à 8, caractérisé en ce que les différentes couches sous forme de bandes sont réunies au cours d'un processus de fabrication continu et continûment comprimées et reliées entre elles en utilisant un dispositif de compression pourvu de moyens de compression par tronçon en épargnant des chambres ou canaux remplis de principes actifs.

10. Procédé selon l'une ou plusieurs des revendications 1 à 9, caractérisé en ce que le façonnage des chambres ou canaux est réalisé à l'aide de poinçons d'impression ou de cylindres d'impression chauffés en utilisant des adhésifs à chaud, ou bien par pression en utilisant des adhésifs sensibles à la pression à l'aide de poinçons d'impression ou de cylindres d'impression non chauffés.

11. Procédé selon l'une ou plusieurs des revendications 1 à 10, caractérisé en ce qu'une bande d'une feuille support conventionnelle contenant des principes actifs est utilisée comme couche supérieure, un adhésif étant appliqué sur celle-ci, qui se laisse étaler à l'état chaud, de préférence à une température comprise entre 50 et 100°C, mais qui durcit et ne colle plus au-dessous de cette température.

12. Procédé selon la revendication 11, caractérisé en ce que la couche supérieure de même que la couche inférieure resp. les bandes correspondantes sont pourvues d'un adhésif thermofusible.

13. Procédé selon l'une des revendications 1 à 12, caractérisé en ce que l'adhésif, au lieu d'être appliqué sur toute la surface de l'une ou des deux couches resp. bandes, est appliqué en forme de réseau, de lignes ou de points, en particulier sur la couche à membrane inférieure.

14. Procédé selon l'une ou plusieurs des revendications 1 à 13, caractérisé en ce que le réchauffement de l'adhésif thermofusible est obtenu par un effet énergétique, par exemple par rayonnement micro-ondes, par rayons infrarouges, par laser à micro-ondes, aux rayons infrarouges ou aux rayons X ou par une combinaison de tels rayonnements.

15. Procédé selon l'une ou plusieurs des revendications 1 à 14, caractérisé en ce qu'immédiatement après la liaison par réchauffement de la couche supérieure et de la couche inférieure, le substrat ainsi réalisé est soumis à un refroidissement de préférence rapide notamment des points d'assemblage.

16. Dispositif de fabrication d'un système thérapeutique transdermique plat à chambres multiples resp. à canaux à chambres multiples pour l'administration de principes actifs à la peau, en particulier pour la mise en oeuvre du procédé selon l'invention, le dispositif comportant des moyens permettant de comprimer des endroits séparés ou contigus de la couche supérieure et de la couche inférieure, caractérisé en ce que ces moyens sont réalisés avec des surfaces d'appui, lesquelles correspondent aux surfaces entre les chambres ou canaux contenant des principes actifs et sont réalisés sous forme de poinçons ou de cylindres.

17. Dispositif selon la revendication 16, caractérisé en ce que les surfaces d'appui des poinçons ou des cylindres sont réalisées de façon à pouvoir être chauffées.

18. Dispositif selon la revendication 17, caractérisé en ce qu'un support est associé à un poinçon d'impression ou un cylindre d'impression et en ce que ledit support est de préférence élastique.

19. Dispositif selon l'une des revendications 1 à 18, caractérisé en ce que deux groupes de poinçons d'impression ou de cylindres d'impression sont disposés l'un derrière l'autre dans une ligne de production de préférence continue et présentent des structures d'impression concordantes, le premier groupe étant conçu pour la compression des couches du substrat et le groupe suivant pour la liaison de celles-ci à l'aide de surfaces d'appui chauffées.

20. Dispositif selon l'une des revendications 1 à 19, caractérisé en ce que la ligne de production comporte une station de refroidissement munie, par exemple, d'une soufflante à air chaud pour le refroidissement de préférence rapide de points d'assemblage à chaud.

21. Dispositif selon l'une ou plusieurs des revendications 1 à 20, caractérisé en ce qu'un poinçon d'impression ou un cylindre d'impression est réalisé, à l'exception de ses surfaces d'appui, sous forme de substrats individuels, à l'aide d'outils de poinçonnage et de découpage, pour la confection de bandes.

22. Dispositif selon l'une ou plusieurs des revendications 1 à 21, caractérisé en ce que celui-ci comporte des moyens permettant la mise sous vide d'un poinçon d'impression ou d'un cylindre de poinçon.

23. Dispositif selon l'une ou plusieurs des revendications 1 à 22, caractérisé en ce que chaque fois deux poinçons d'impression ou cylindres d'impression sont disposés de manière à pouvoir se déplacer l'un vers l'autre par le haut ou par le bas et réalisés de manière à coopérer l'un avec l'autre sous pression.
